# EUROPEAN PATENT SPECIFICATION

(11) **EP 1 928 869 B1**
(45) Date of publication and mention of the grant of the patent: **17.03.2010**
(21) Application number: 06765302.2
(22) Date of filing: 18.08.2006
(51) Int. Cl.: C07D 417/12, C07D 275/02

(54) **CHEMICAL PROCESS**
CHEMISCHES VERFAHREN
PROCÉDÉ CHIMIQUE

(30) Priority: 19.08.2005 GB 0517050
(43) Date of publication of application: 11.06.2008
(73) Proprietor: Syngenta Participations AG, 4058 Basel (CH)
(72) Inventor: FAROOQ, Saleem, 4422 Arlsdorf (CH); HALL, Roger, Graham, 4058 Basel (CH)
(74) Representative: Ward, Steven Paul
(86) International application number: PCT/GB2006/003101
(87) International publication number: WO 2007/020460

(56) References cited:
- WO-A-01/55138
- WO-A-02/059120
- WO-A-03/011861
- CHANG J ET AL: "Synthesis of 2-arylbenzoxazoles via DDQ promoted oxidative cyclization of phenolic Schiff bases-a solution-phase strategy for library synthesis" TETRAHEDRON LETTERS, ELSEVIER, AMSTERDAM, NL, vol. 43, no. 6, 4 February 2002 (2002-02-04), pages 951-954, XP004333934 ISSN: 0040-4039

## Description

The present invention relates to an improved process for making azole derivatives useful as insecticidal, acaricidal, molluscicidal and nematicidal compoonds.

Azole derivatives with useful insecticidal properties are disclosed in WO00/06566, WO00/63207, WO01/55144 and WO03/011861. The applicants have found a method of making the compounds in improved yield. There is therefore provided a process for the preparation of compounds of formula (I) 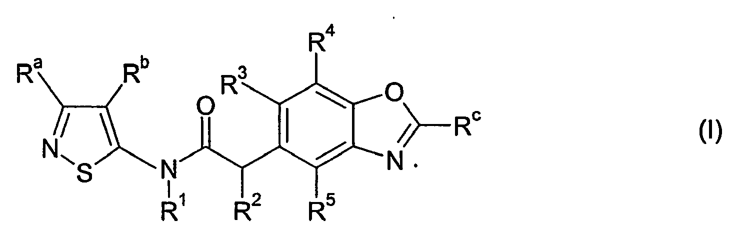 wherein R^{a} is C₁₋₃ alkyl; R^{b} is halogen; R^{c} is C₁₋₆ alkoxy(C₁₋₆)alkyl, C₁₋₆ haloalkyl, C₁₋₆ alkyl or C₁₋₆ alkoxy, or is a group 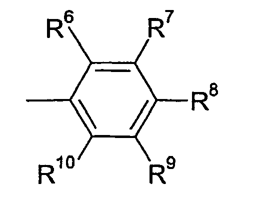 R¹ is hydrogen, C₁₋₂ alkyl, (C₁₋₆) alkoxymethyl or propargyl; R² is hydrogen, methyl or fluoro; R³, R⁴ and R⁵ are, independently, hydrogen, halogen, C₁₋₂ alkyl, C₁₋₂ alkoxy or C₁₋₂ haloalkyl; R⁶ and R¹⁰ are, independently, hydrogen, halogen, C₁₋₃ alkyl, C₁₋₂ haloalkyl, C₁₋₂ alkoxy, nitro, cyano, C₁₋₂ haloalkoxy, C₁₋₈ alkylthio, C₁₋₆ akylsulfinyl, C₁₋₆ alkylsulfonyl, amino, C₁₋₃ alkylamino or di(C₁₋₃)alkylamino; R⁷, R⁸ and R⁹ are, independently, hydrogen, halogen, C₁₋₆ alkyl, C₂₋₆ alkenyl, C₂₋₆ alkynyl, C₁₋₆ haloalkyl, C₁₋₆ alkoxy(C₁₋₆)alkyl, C₁₋₆ alkoxy, C₁₋₆ alkoxy(C₁₋₆)alkoxy, C₂₋₆ alkynyloxy, C₃₋₆ cycloalkyl, nitro, cyano, C₁₋₆ haloalkoxy, C₂₋₆ haloalkenyloxy, S(O)ₚR¹¹, OSO₂R¹², NR¹³SO₂R¹⁴, NR¹⁵R¹⁶, NR¹⁷COR¹⁸, COR¹⁹, SiR²⁰R²¹R²², SCN, optionally substituted aryl or optionally substituted heteroaryl or optionally substituted heterocyclyl;
R¹¹, R¹² and R¹⁴ are, independently, C₁₋₆ alkyl, C₁₋₆ haloalkyl or optionally substitituted aryl; R¹³ and R¹⁷ are, independently, hydrogen or C₁₋₂ alkyl; R¹⁵ and R¹⁶ are, independently, hydrogen or C₁₋₃ alkyl; or R¹⁵ and R¹⁶ together with the N atom to which they are attached form a five or six-membered optionally substituted heterocyclic ring which may contain a further heteroatom selected from O and S; R¹⁸ and R¹⁹ are, independently, hydrogen, C₁₋₆ alkyl, C₁₋₆ alkoxy, optionally substituted aryl, optionally substituted heteroaryl or NR²³R²⁴; R²⁰, R²¹ and R²² are, independently, C₁₋₄ alkyl or aryl; R²³ and R²⁴ are, independently, hydrogen or C₁₋₃ alkyl; or R²³ and R²⁴ together with the N atom to which they are attached form a five or six-membered optionally substituted heterocyclic ring which may contain a further heteroatom selected from O and S; and p is 0, 1 or 2, the process comprising reacting a formula of compound II 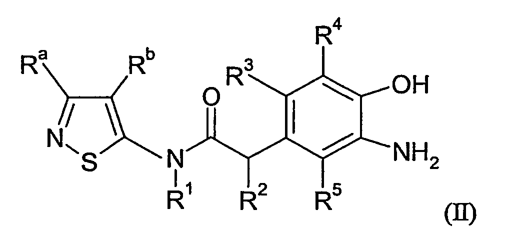 where R^{a}, R^{b}, R¹, R², R³, R⁴ and R⁵ are as defined in relation to formula (I) with a compound of formula III

R^{c}CHO (III)

where R^{c} is as defined in relation to formula (I) and an oxidising agent.

The reaction proceeds via compounds of formula (IV) as shown below.

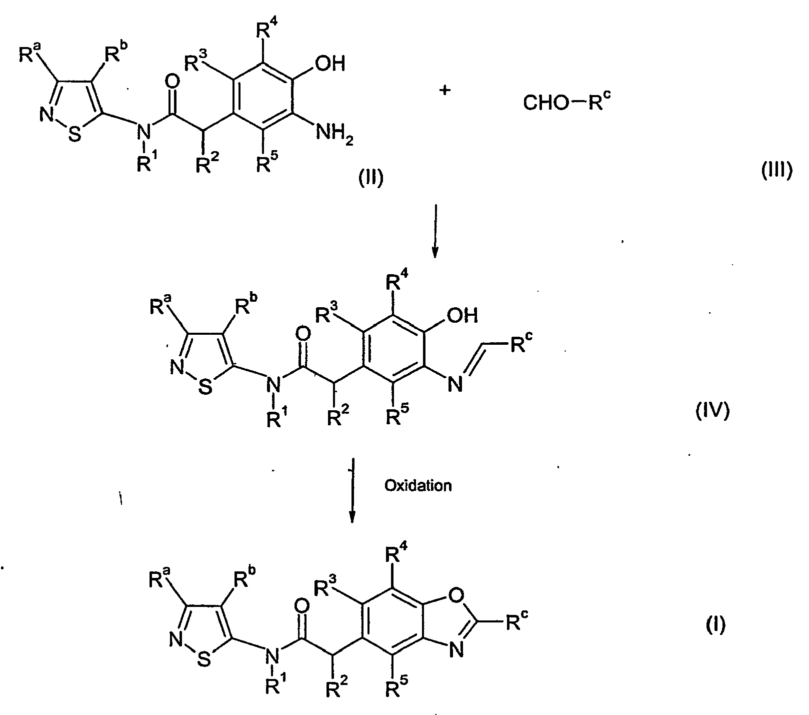

The intermediate compound of formula (IV) may, depending on the exact reaction conditions and the value of R^{c}, be formed either as a Schiff base as shown or in the form of a cyclic amide of formula (IV')

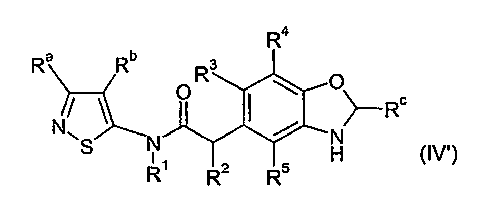

In a preferred embodiment of the invention the reactions are performed stepwise so that the intermediate of formula (IV) or (IV') is formed first and the intermediate is then converted into a compound of formula I by treatment with the oxidising agent.

The intermediate of formula (IV) or (IV') may be isolated or the process can be performed without isolation of the intermediate.

Certain compounds of formula (IV) and (IV') are novel and as such form a further aspect of the invention.

Suitable oxidising agents for use in the reaction include UV light; air; oxygen; bromine; acetates such as lead tetraacetate, iodosobenzenediacetate and manganese triacetate; perchlorates such as sodium perchlorate and thianthrene cation radical perchlorate; manganates such as barium manganate; peroxides such as nickel peroxide; oxides such as manganese dioxide; dichloro-5,6-dicyano-1,4-benzoquinone and N-bromosuccinimide.

Preferred oxidising agents are air, oxygen, bromine, acetates such as lead tetraacetate, perchlorates such as sodium perchlorate and N-bromosuccinimide.

The oxidation reaction is suitably performed at a temperature of 0 to 100°C, preferably 10 to 50°C, more preferably at 15 to 30 °C.

The oxidation reaction is preferably performed in a solvent. Preferred solvents are acids, preferably carboxylic acids for example acetic acid or halogenated alkanes such as carbon tetrachloride.

The oxidation reaction may optionally be performed in the presence of radical initiators. Suitable free-radical initiators are well known to the person skilled in the art and include for example aroyl peroxides such as dibenzoyl peroxide, and azo compounds such as azobisisobutyronitrile, which is particularly preferred.

The addition of the aldehyde to a compound of formula II may suitably be performed at 20-150°C. The reaction is suitably performed in any suitable solvent such as toluene, xylene etc.

Each alkyl moiety is a straight or branched chain and is, for example, methyl, ethyl, *n*-propyl, *n*-butyl, *n*-pentyl, *n*-hexyl, *iso*-propyl, *n*-butyl, *sec*-butyl, *iso*-butyl, *tert*-butyl or *neo*-pentyl.

Halogen is fluorine, chlorine, bromine or iodine.

Haloalkyl groups are alkyl groups which are substituted with one or more of the same or different halogen atoms and are, for example, CF₃, CF₂Cl, CF₃CH₂ or CHF₂CH₂.

Alkenyl and alkynyl moieties can be in the form of straight or branched chains. The alkenyl moieties, where appropriate, can be of either the (E)- or (Z)-configuration. Examples are vinyl, allyl, ethynyl and propargyl.

Haloalkenyl moieties are alkyl moieties which are substituted with one or more of the same or different halogen atoms, an example being CH₂CH=CCl₂.

Aryl includes naphthyl, anthracyl, fluorenyl and indenyl but is preferably phenyl.

The term heteroaryl refers to an aromatic ring containing up to 10 atoms including one or more heteroatoms (preferably one or two heteroatoms) selected from O, S and N. Examples of such rings include pyridine, pyrimidine, furan, quinoline, quinazoline, pyrazole, thiophene, thiazole, oxazole and isoxazole.

The terms heterocycle and heterocyclyl refer to a non-aromatic ring containing up to 10 atoms including one or more (preferably one or two) heteroatoms selected from O, S and N. Examples of such rings include 1,3-dioxolane, tetrahydrofuran and morpholine.

Cycloalkyl includes cyclopropyl, cyclopentyl and cyclohexyl.

When present, the optional substituents on aryl, heteroaryl or heterocyclyl are selected, independently, from hydrogen, halogen, C₁₋₆ alkyl, C₂₋₆ alkenyl, C₂₋₆ alkynyl, C₁₋₆ haloalkyl, C₁₋₆ alkoxy(C₁₋₆)alkyl, C₁₋₆ alkoxy, C₃₋₆ cycloalkyl, nitro, cyano, C₁₋₆ haloalkoxy, C₁₋₂ alkylthio, SO₂CH₃, SO₂CH₂CH₃, OSO₂CH₃ and SCN.

It is to be understood that dialkylamino substituents include those where the dialkyl groups together with the N atom to which they are attached form a five, six or seven-membered heterocyclic ring which may contain one or two further heteroatoms selected from O, N or S and which is optionally substituted by one or two independently selected (C₁₋₆)alkyl groups. When heterocyclic rings are formed by joining two groups on an N atom, the resulting rings are suitably pyrrolidine, piperidine, thiomorpholine and morpholine each of which may be substituted by one or two independently selected (C₁₋₆) alkyl groups.

Preferred groups for R^{a}, R^{b}, R^{c}, R¹, R², R³, R⁴ and R⁵ in any combination thereof are set out below.

Preferably R^{a} is methyl or ethyl.

It is peferred that R^{b} is bromo or chloro, especially chloro.

The group R^{c} is preferably is a group 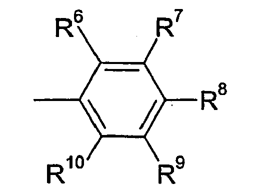 or is C₁₋₆ alkyl or is C₁₋₆ haloalkyl.

More preferably R^{c} is C₁₋₆ alkyl or C₁₋₆ haloalkyl, more especially C₁₋₃ haloalkyl.

Preferably R¹ is hydrogen, C₁₋₂ alkyl or (C₁₋₆) alkoxymethyl.

It is more preferred that R¹ is hydrogen, ethyl, CH₂OCH₃ or CH₂OC₂H₅.

Yet more preferably R¹ is hydrogen, ethyl or CH₂OC₂H₅.

It is even more preferred that R¹ is hydrogen or CH₂OC₂H₅, especially hydrogen.

Preferably R² is hydrogen or fluoro.

In one aspect of the invention, it is preferred that R² is fluouro.

Preferably R³, R⁴ and R⁵ are each, independently, hydrogen or halogen.

It is preferred that R³ is hydrogen or fluorine.

More preferably R³ is hydrogen.

It is preferred that R⁴ is hydrogen or fluorine.

More preferably R⁴ is hydrogen.

It is preferred that R⁵ is hydrogen or fluorine.

More preferably R⁵ is hydrogen.

It is preferred that R⁷, R⁸ and R⁹ are each, independently, hydrogen, halogen, C₁₋₆ alkyl, C₁₋₆ haloalkyl, C₁₋₆ alkoxy, C₁₋₆ akoxy(C₁₋₆)akoxy, C₂₋₆ alkynyloxy, nitro, cyano, C₁₋₆ alkylthio, C₁₋₆ alkylsulfonyl or C₂₋₆ haloalkenyloxy.

It is preferred that R⁷ is hydrogen, halogen, C₁₋₆ alkyl, C₁₋₆ alkoxy(C₁₋₆)alkoxy, nitro or cyano.

More preferably R⁷ is hydrogen, chlorine, fluorine, methyl, OC₂H₄OCH₃, nitro or cyano.

It is even more preferred that R⁷ is hydrogen or chlorine.

It is yet more preferred that R⁷ is hydrogen.

It is preferred that R⁸ is hydrogen, halogen, C₁₋₆ haloalkyl, C₁₋₆ alkoxy, C₁₋₆ alkoxy(C₁₋₆)alkoxy, C₂₋₆ alkynyloxy, cyano, C₁₋₆ alkylsulfonyl or C₂₋₆ haloalkenyloxy.

More preferably R⁸ is hydrogen, chlorine, fluorine, bromine, CF₃, ethoxy, OC₂H₄OCH₃, OCH₂C ≡CH, cyano, SO₂CH₃ or OCH₂CH=CCl₂.

It is even more preferred that R⁸ is hydrogen, chlorine, CN, CF₃ or SO₂CH₃.

Yet more preferably R⁸ is hydrogen.

It is preferred that R⁹ is hydrogen, halogen or C₁₋₆ alkylthio.

More preferably R⁹ is hydrogen, chlorine, fluorine, iodine or SCH₃.

It is even more preferred that R⁹ is hydrogen, chlorine or fluorine.

Yet more preferably R⁹ is hydrogen.

It is preferred that R⁶ and R¹⁰ are, independently, hydrogen, halogen, C₁₋₃ alkyl, C₁₋₂ haloalkyl, C₁₋₂ alkoxy, nitro, cyano, C₁₋₂ haloalkoxy, C₁₋₈ alkylthio or C₁₋₆ alkylsulfinyl, C₁₋₆ alkylsulfonyl; provided that at least one of R⁶ and R¹⁰ is not hydrogen.

In one aspect of the invention, it is preferred that R⁶ and R¹⁰ are, independently, hydrogen, halogen, C₁₋₃ alkyl, C₁₋₂ haloalkyl, C₁₋₂ alkoxy, nitro, cyano, C₁₋₂ haloalkoxy or C₁₋₂ alkylthio, provided that at least one of R⁶ and R¹⁰ is not hydrogen.

It is more preferred that R⁶ is hydrogen, methyl, chlorine, fluorine or bromine and R¹⁰ is hydrogen, methyl, chlorine, fluorine, OCH₃, SCH₃, CF₃ or nitro, provided that at least one of R⁶ and R¹⁰ is not hydrogen.

It is still more preferred that R⁶ is hydrogen, chlorine, fluorine or bromine and R¹⁰ is hydrogen, chlorine, fluorine, OCH₃, SCH₃, CF₃ or nitro, provided that at least one of R⁶ and R¹⁰ is not hydrogen.

Even more preferably R⁶ is hydrogen, chlorine, fluorine or bromine and R¹⁰ is chlorine, fluorine or bromine.

It is most preferred that when R⁶ is hydrogen, R¹⁰ is fluorine, chlorine or bromine and that when R⁶ is chlorine or fluorine, R¹⁰ is fluorine.

The invention is illustrated by the following Examples:

### EXAMPLE 1

### This Example illustrates the preparation of N-(4-chloro-3-ethylisothiazol-5-yl)-2-[2-(2,6-dichlorophenyl)benzoxazol-5-yl] propionamide.

### Step a)

N-(4-chloro-3-ethylisothiazol-5-yl)-2-(3-amino-4-hydroxyphenyl)propionamide prepared as described in WO03/011861 (20.0 gm, 0.06 mole) was dissolved in toluene (520 ml) and 2,6-dichlorobenzaldehyde (12.0 gm, 0.068 mole) was added. The resulting mixture was refluxed for 12 hours. The reaction was allowed to cool, and the solvent evaporated under reduced pressure, leaving the intermediate as pale yellow crystals (32.4 gm).

### Step b)

The intermediate from step a) was suspended in acetic acid (400 ml) to which was added Lead tetraacetate (33.6 gm, 0.076 mole) in portions. During the addition of the oxidant, external cooling with an ice bath was employed to maintain the exotherm at around room temperature. After stirring at room temperature for 3 hours, the reaction mixture was poured onto stirred ice-water (1.61) and then extracted three times with ethyl acetate. The organic phase was then washed four times with water, twice with a saturated sodium chloride solution, and finally dried over sodium sulphate. After filtration, and removal of solvent under reduced pressure, the resulting product was recrystallised from ether to give N-(4-chloro-3-ethylisothiazol-5-yl)-2-[2-(2,6-dichlorophenyl)benoxazol-5-yl] propionamide.8.5 gm. The solvent was removed from the filtrate, and the residue was purified by chromatography on silica gel, using a mixture of ethyl acetate:hexane 1:2. A further 9.1 gm product was obtained, giving a total yield of 17.6 gm N-(4-chloro-3-ethylisothiazol-5-yl)-2-[2-(2,6-dichlorophenyl)benzoxazol-5-yl] propionamide with melting point 180-183°C.

### EXAMPLE 2

### Preparation of N-(4-chloro-3-ethyl-5-isothiazolyl)-2-(heptafluoropropyl)-5-benzoxazoleacetamide

### Step a)

N-(4-chloro-3-ethylisothiazol-5-yl)-2-(3-amino-4-hydroxyphenyl)acetamide prepared as described in WO03/011861 (3.1 gm, 10.0 mmol) was dissolved in toluene (100ml) and heptafluorobutyraldehyde hydrate (2.6 gm, 11.2 mmol) was added followed by para-toluenesulphunic acid (0.19 gm, 1 mmol) at room temperature. The resulting mixture was fitted with a Dean-Stark apparatus and refluxed for 12 hours allowing water to be separated. The reaction was allowed to cool, a small amount of a gummy material removed by filtration and the filtrate was concentrated under reduced pressure. The residue was purified by chrmoatorgraphy using a mixture of hexane:ethyl acetate (3:1.8). After chromatography the product was further purified by stirring in hot hexane (20 ml) adding ethyl acetate (10 ml), cooling to -20°C and isolating the product by filtration to give white crystals with melting point 122-124°C.

### Step b)

The product from step a) (0.393 gm, 0.8 mmol) was suspended in carbon tetrachloride (20 ml) and N-Iodosuccinimide (0.36 gm, 1.6 mmol) was added. The resulting mixture was heated to reflux for 1 hour. The reaction mixture was allowed to cool and stirred with 10% sodiumsulphite solution (20 ml) to destroy any iodine formed. The organic layer was separated, washed twice with dilute saline solution and then dried over sodium sulphate. After removal of the solvent the residue was purified by chromatography using a mixture of hexane:ethyl acetate (3:1) to give the product as white crystals. 1H nmr: δ 1.3 (3H, t), 2.75 (2H, q), 4.0 (2H, s), 7.55 (1H, d), 7.7 (1H, d), 7.9 (1H, s), 8.2 (1H, br.s).

### EXAMPLE 2A

### Preparation of N-(4-chloro-3-ethyl-5-isothiazolyl)-2-(heptafluoropropyl)-5-benzoxazoleacetamide

The product from Example 2, step a) (0.393 gm, 0.8 mmol) was suspended in acetonitrile (10 ml) and (diacetoxyiodo)benzene (0.283 gm, 0.88 mmol) was added. The resulting mixture was stirred at room temperature for 0.5 hour. The solvent was removed under reduced pressure and the residue purified by chromatography to give a product identical to that obtained in Example 2, step b).

### EXAMPLE 2B

### Preparation of N-(4-chloro-3-ethyl-5-isothiazolyl)-2-(heptafluoropropyl)-5-Benzoxazoleacetamide

The product from Example 2, step a) (0.393 gm, 0.8 mmol) was suspended in acetic acid (5 ml) and lead tetraacetate (0.391 gm, 0.88 mmol) was added in five portions. The resulting mixture was stirred at room temperature for 3 hours. The mixture was then poured onto ice/water and extracted into an organic phase by washing twice with ethyl acetate (2x 15 ml). The combined organic layer was dried, the solvent removed under reduced pressure and the residue purified by chromatography to give a product identical to that obtained in Example 2, step b).

### EXAMPLE 3

### Preparation of N-(4-chloro-3-methyl-5-isothiazolyl)-2-(heptafluoropropyl)-ά-methyl-5-benzoxazaleacetamide

### Step a)

N-(4-chloro-3-methylisothiazol-5-yl)-2-(3-amino-4-hydroxyphenyl)propionamide prepared as described in WO01/055139 (6.2 gm, 20.0 mmol) was dissolved in toluene (200ml) and heptafluorobutyraldehyde hydrate (5.2 gm, 24 mmol) was added followed by para-toluenesulphunic acid (0.3 gm, 1.7 mmol) at room temperature. The resulting mixture was fitted with a Dean-Stark apparatus and refluxed for 12 hours allowing water to be separated. The reaction was allowed to cool, a small amount of a gummy material removed by filtration and the filtrate was concentrated under reduced pressure. The residue was purified by chrmoatorgraphy using a mixture of hexane:ethyl acetate (1:1). The product was obtained as white crystals with melting point 140 °C.

### Step b)

The product from step a) (0.492 gm, 1.0 mmol) was suspended in carbon tetrachloride (25 ml) and N-Iodosuccinimide (0.45 gm, 2.0 mmol) was added. The resulting mixture was heated to reflux for 1 hour. The reaction mixture was allowed to cool and stirred with 10% sodiumsulphite solution (20 ml) to destroy any iodine formed. The organic layer was separated, washed twice with dilute saline solution and then dried over sodium sulphate. After removal of the solvent the residue was purified by chromatography using a mixture of hexane:ethyl acetate (3:1) to give the product as white crystals. 1H nmr: δ 1.7 (3H, d), 2.4 (3H, s), 4.0 (1H, q), 7.55 (1H, d), 7.7 (1H, d), 7.9 (1H, s), 8.0 (1H, br.s).

### EXAMPLE 3A

### Preparation of N-(4-chloro-3-methyl-5-isothiazolyl)-2-(heptafluoropropyl)-άmethyl-5-benzoxazoleacetamide

The product from Example 3, step a) (0.492 gm, 1.0 mmol) was suspended in acetonitrile (10 ml) and (diacetoxyiodo)benzene (0.354 gm, 1.1 mmol) was added. The resulting mixture was stirred at room temperature for 0.5 hour. The solvent was removed under reduced pressure and the residue purified by chromatography to give a product identical to that obtained in Example 3, step b).

### EXAMPLE 3B

### Preparation of N-(4-chloro-3-methyl-5-isothiazolyl)-2-(heptafluoropropyl)-ά-methyl-5-benzoxazoleacetamide

The product from Example 3, step a) (0.492 gm, 1.0 mmol) was suspended in acetic acid (6 nil) and lead tetraacetate (0.488 gm, 1.1 mmol) was added in five portions. The resulting mixture was stirred at room temperature for 3 hours. The mixture was then poured onto ice/water and extracted into an organic phase by washing twice with ethyl acetate (2x 15 ml). The combined organic layer was dried, the solvent removed under reduced pressure and the residue purified by chromatography to give a product identical to that obtained in Example 3, step b).

## Claims

1. A process for the preparation of a compound of formula (I) 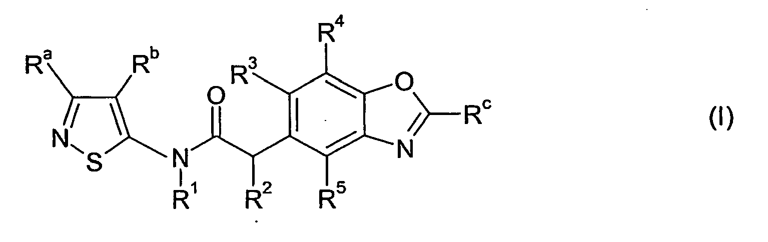 wherein R^{a} is C₁₋₃ alkyl; R^{b} is halogen; R^{c} is C₁₋₆ alkoxy(C₁₋₆)alkyl, C₁₋₆ haloalkyl, C₁₋₆ alkyl or C₁₋₆ alkoxy, or is a group 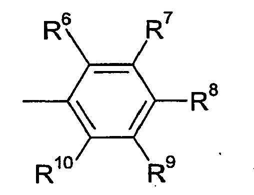 R¹ is hydrogen, C₁₋₂ alkyl, (C₁₋₆)alkoxymethyl or propargyl; R² is hydrogen, methyl or fluoro; R³, R⁴ and R⁵ are, independently, hydrogen, halogen, C₁₋₂ alkyl, C₁₋₂ alkoxy or C₁₋₂ haloalkyl; R⁶ and R¹⁰ are, independently, hydrogen, halogen, C₁₋₃ alkyl, C₁₋₂ haloalkyl, C₁₋₂ alkoxy, nitro, cyano, C₁₋₂ haloalkoxy, C₁₋₈ alkylthio, C₁₋₆ alkylsulfinyl, C₁₋₆ alkylsulfonyl, amino, C₁₋₃ alkylamino or di(C₁₋₃)alkylamino; R⁷, R⁸ and R⁹ are, independently, hydrogen, halogen, C₁₋₆ alkyl, C₂₋₆ alkenyl, C₂₋₆ alkynyl, C₁₋₆ haloalkyl, C₁₋₆ alkoxy(C₁₋₆)alkyl, C₁₋₆ alkoxy, C₁₋₆ alkoxy(C₁₋₆)alkoxy, C₂₋₆ alkynyloxy, C₃₋₆ cycloalkyl, nitro, cyano, C₁₋₆ haloalkoxy, C₂₋₆ haloalkenyloxy, S(O)ₚR¹¹, OSO₂R¹², NR¹³SO₂R¹⁴, NR¹⁵R¹⁶, NR¹⁷COR¹⁸, COR¹⁹, SiR²⁰R²¹R²², SCN, optionally substituted aryl or optionally substituted heteroaryl or optionally substituted heterocyclyl;
R¹¹, R¹² and R¹⁴ are, independently, C₁₋₆ alkyl, C₁₋₆ haloalkyl or optionally substitituted aryl; R¹³ and R¹⁷ are, independently, hydrogen or C₁₋₂ alkyl; R¹⁵ and R¹⁶ are, independently, hydrogen or C₁₋₃ alkyl; or R¹⁵ and R¹⁶ together with the N atom to which they are attached form a five or six-membered optionally substituted heterocyclic ring which may contain a further heteroatom selected from O and S; R¹⁸ and R¹⁹ are, independently, hydrogen, C₁₋₆ alkyl, C₁₋₆ alkoxy, optionally substituted aryl, optionally substituted heteroaryl or NR²³R²⁴; R²⁰, R²¹ and R²² are, independently, C₁₋₄ alkyl or aryl;
R²³ and R²⁴ are, independently, hydrogen or C₁₋₃ alkyl; or R²³ and R²⁴ together with the N atom to which they are attached form a five or six-membered optionally substituted heterocyclic ring which may contain a further heteroatom selected from O and S; and p is 0, 1 or 2 the process comprising reacting a formula of compound II 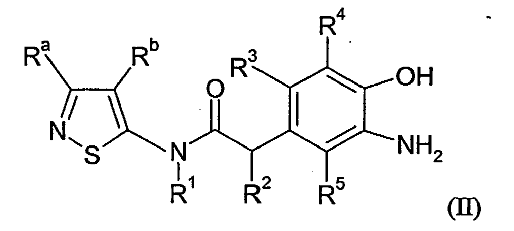 where R^{a}, R^{b}, R¹, R², R³, R⁴ and R⁵ are as defined in relation to formula (I) with a compound of formula III
R^{c}CHO (III)
where R^{c} is as defined in relation to formula (I) and an oxidising agent.

2. A process as claimed in claim 1 where R^{c} is a group 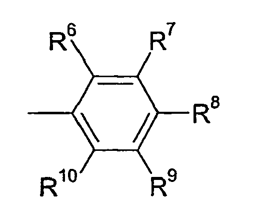 wherein R⁶ and R¹⁰ are, independently, hydrogen, halogen, C₁₋₃ alkyl, C₁₋₂ haloalkyl, C₁₋₂ alkoxy, nitro, cyano, C₁₋₂ haloalkoxy, C₁₋₂alkylthio, amino, C₁₋₃ alkylamino or di(C₁₋₃)alkylamino, provided that at least one of R⁶ and R¹⁰ is not hydrogen; and R⁷, R⁸ and R⁹ are, independently, hydrogen, halogen, C₁₋₆ alkyl, C₂₋₆ alkenyl, C₂₋₆ alkynyl, C₁₋₆ haloalkyl, C₁₋₆ alkoxy(C₁₋₆)alkyl, C₁₋₆ alkoxy, C₃₋₆ cycloalkyl, nitro, cyano, C₁₋₆ haloalkoxy, S(O)ₚR¹¹, OSO₂R¹², NR¹³SO₂R¹⁴, NR¹⁵R¹⁶, NR¹⁷COR¹⁸, COR¹⁹, SiR²⁰R²¹R²², SCN, optionally substituted aryl or optionally substituted heteroaryl.

3. A process as claimed in claim 1 where R^{c} is C₁₋₆ alkyl or C₁₋₆ haloalkyl.

4. A process as claimed in any of the preceding claims where R¹ is hydrogen, C₁₋₂ alkyl or (C₁₋₆) alkoxymethyl.

5. A process as claimed in any of the preceding claims where R² is hydrogen or fluouro.

6. A process as claimed in any of the preceding claims where R³, R⁴ and R⁵ are each, independently, hydrogen or halogen.

7. A process for the preparation of a compound of formula I as defined in claim 1 which process comprises reacting a compound of formula II as defined in claim 1 with a compound of formula III as defined in claim 1 to produce a compound of formula (IV) or formula (IV') 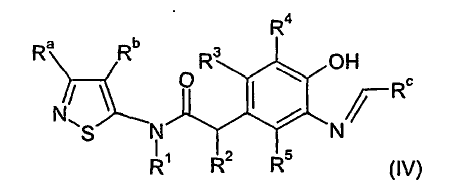 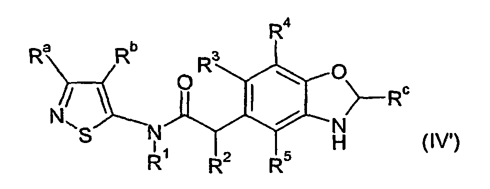 wherein R^{a} is C₁₋₃ alkyl; R^{b} is halogen; R^{c} is C₁₋₆ alkoxy(C₁₋₆)alkyl, C₁₋₆ haloalkyl, C₁₋₆ alkyl or C₁₋₆ alkoxy, or is a group 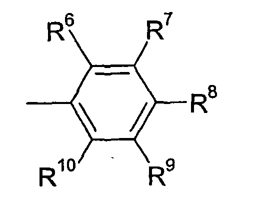 R¹ is hydrogen, C₁₋₂ alkyl, (C₁₋₆)alkoxymethyl or propargyl; R² is hydrogen, methyl or fluoro; R³, R⁴ and R⁵ are, independently, hydrogen, halogen, C₁₋₂ alkyl, C₁₋₂ alkoxy or C₁₋₂ haloalkyl; R⁶ and R¹⁰ are, independently, hydrogen, halogen, C₁₋₃ alkyl, C₁₋₂ haloalkyl, C₁₋₂ alkoxy, nitro, cyano, C₁₋₂ haloalkoxy, C₁₋₈ alkylthio, C₁₋₆ alkylsulfinyl, C₁₋₆ alkylsulfonyl, amino, C₁₋₃ alkylamino or di(C₁₋₃)alkylamino; R⁷, R⁸ and R⁹ are, independently, hydrogen, halogen, C₁₋₆ alkyl, C₂₋₆ alkenyl, C₂₋₆ alkynyl, C₁₋₆ haloalkyl, C₁₋₆ alkoxy(C₁₋₆)alkyl, C₁₋₆ alkoxy, C₁₋₆ alkoxy(C₁₋₆)alkoxy, C₂₋₆ alkynyloxy, C₃₋₆ cycloalkyl, nitro, cyano, C₁₋₆ haloalkoxy, C₂₋₆ haloalkenyloxy, S(O)ₚR¹¹, OSO₂R¹², NR¹³SO₂R¹⁴, NR¹⁵R¹⁶, NR¹⁷COR¹⁸, COR¹⁹, SiR²⁰R²¹R²², SCN, optionally substituted aryl or optionally substituted heteroaryl or optionally substituted heterocyclyl;
R¹¹, R¹² and R¹⁴ are, independently, C₁₋₆ alkyl, C₁₋₆ haloalkyl or optionally substitituted aryl; R¹³ and R¹⁷ are, independently, hydrogen or C₁₋₂ alkyl; R¹⁵ and R¹⁶ are, independently, hydrogen or C₁₋₃ alkyl; or R¹⁵ and R¹⁶ together with the N atom to which they are attached form a five or six-membered optionally substituted heterocyclic ring which may contain a further heteroatom selected from O and S; R¹⁸ and R¹⁹ are, independently, hydrogen, C₁₋₆ alkyl, C₁₋₆ alkoxy, optionally substituted aryl, optionally substituted heteroaryl or NR²³R²⁴; R²⁰, R²¹ and R²² are, independently, C₁₋₄ alkyl or aryl;
R²³ and R²⁴ are, independently, hydrogen or C₁₋₃ alkyl; or R²³ and R²⁴ together with the N atom to which they are attached form a five or six-membered optionally substituted heterocyclic ring which may contain a further heteroatom selected from O and S; and p is 0, 1 or 2 and reacting the compound of formual (IV) or (IV') with an oxidising agent.

8. A process according to any preceeding claim where the oxidising agent is UV light; air; oxygen; bromine; acetates such as lead tetraacetate, iodosobenzenediacetate and manganese triacetate; perchlorates such as sodium perchlorate and thianthrene cation radical perchlorate; manganates such as barium manganate; peroxides such as nickel peroxide; oxides such as manganese dioxide; dichloro-5,6-dicyano-1,4-benzoquinone and N-bromosuccinimide.

9. A compound of formula IV or formula IV' 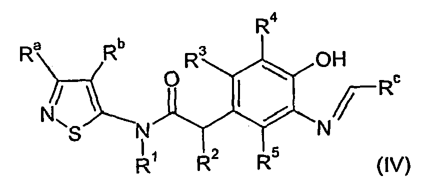 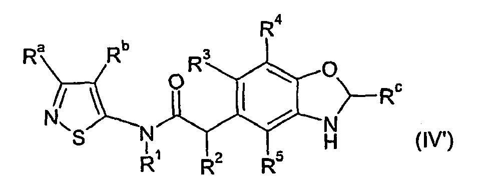 wherein R^{a} is C₁₋₃ alkyl; R^{b} is halogen; R^{c} is C₁₋₆ alkoxy(C₁₋₆)alkyl, C₁₋₆ haloalkyl, C₁₋₆ alkyl or C₁₋₆ alkoxy, or is a group 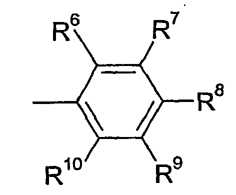 R¹ is hydrogen, C₁₋₂ alkyl, (Cₗ₋₆)alkoxymethyl or propargyl; R² is hydrogen, methyl or fluoro; R³, R⁴ and R⁵ are, independently, hydrogen, halogen, C₁₋₂ alkyl, C₁₋₂ alkoxy or C₁₋₂ haloalkyl; R⁶ and R¹⁰ are, independently, hydrogen, halogen, C₁₋₃ alkyl, C₁₋₂ haloalkyl, C₁₋₂ alkoxy, nitro, cyano, C₁₋₂ haloalkoxy, C₁₋₈ alkylthio, C₁₋₆ alkylsulfinyl, C₁₋₆ alkylsulfonyl, amino, C₁₋₃ alkylamino or di(C₁₋₃)alkylamino; R⁷, R⁸ and R⁹ are, independently, hydrogen, halogen, C₁₋₆ alkyl, C₂₋₆ alkenyl, C₂₋₆ alkynyl, C₁₋₆ haloalkyl, C₁₋₆ alkoxy(C₁₋₆)alkyl, C₁₋₆ alkoxy, C₁₋₆ alkoxy(C₁₋₆)alkoxy, C₂₋₆ alkynyloxy, C₃₋₆ cycloalkyl, nitro, cyano, C₁₋₆ haloalkoxy, C₂₋₆ haloalkenyloxy, S(O)ₚR¹¹, OSO₂R¹², NR¹³SO₂R¹⁴, NR¹⁵R¹⁶, NR¹⁷COR¹⁸, COR¹⁹, SiR²⁰R²¹R²², SCN, optionally substituted aryl or optionally substituted heteroaryl or optionally substituted heterocyclyl;
R¹¹, R¹² and R¹⁴ are, independently, C₁₋₆ alkyl, C₁₋₆ haloalkyl or optionally substitituted aryl; R¹³ and R¹⁷ are, independently, hydrogen or C₁₋₂ alkyl; R¹⁵ and R¹⁶ are, independently, hydrogen or C₁₋₃ alkyl; or R¹⁵ and R¹⁶ together with the N atom to which they are attached form a five or six-membered optionally substituted heterocyclic ring which may contain a further heteroatom selected from O and S; R¹⁸ and R¹⁹ are, independently, hydrogen, C₁₋₆ alkyl, C₁₋₆ alkoxy, optionally substituted aryl, optionally substituted heteroaryl or NR²³R²⁴; R²⁰, R²¹ and R²² are, independently, C₁₋₄ alkyl or aryl;
R²³ and R²⁴ are, independently, hydrogen or C₁₋₃ alkyl; or R²³ and R²⁴ together with the N atom to which they are attached form a five or six-membered optionally substituted heterocyclic ring which may contain a further heteroatom selected from O and S; and p is 0, 1 or 2.

## Patentansprüche

1. Verfahren zur Herstellung einer Verbindung der Formel (I) : 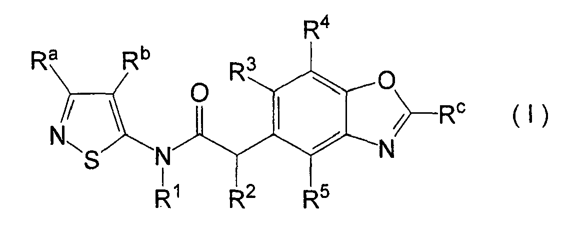 worin R^{a} C₁₋₃-Alkyl ist; R^{b} Halogen ist; R^{c} C₁₋₆-Alkoxy-(C₁₋₆)-alkyl, C₁₋₆-Halogenalkyl, C₁₋₆-Alkyl oder C₁₋₆-Alkoxy oder eine Gruppe 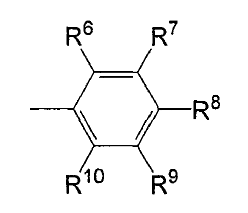 ist; R¹ Wasserstoff, C₁₋₂-Alkyl, (C₁₋₆)-Alkoxymethyl oder Propargyl ist; R² Wasserstoff, Methyl oder Fluor ist; R³, R⁴ und R⁵ unabhängig voneinander Wasserstoff, Halogen, C₁₋₂-Alkyl, C₁₋₂-Alkoxy oder C₁₋₂-Halogenalkyl sind; R⁶ und R¹⁰ unabhängig voneinander Wasserstoff, Halogen, C₁₋₃-Alkyl, C₁₋₂-Halogenalkyl, C₁₋₂-Alkoxy, Nitro, Cyano, C₁₋₂-Halogenalkoxy, C₁₋₈-Alkylthio, C₁₋₆-Alkylsulfinyl, C₁₋₆-Alkylsulfonyl, Amino, C₁₋₃-Alkylamino oder Di-(C₁₋₃)-alkylamino sind; R⁷, R⁸ und R⁹ unabhängig voneinander Wasserstoff, Halogen, C₁₋₆-Alkyl, C₂₋₆-Alkenyl, C₂₋₆-Alkinyl, C₁₋₆-Halogenalkyl, C₁₋₆-Alkoxy-(C₁₋₆)-alkyl, C₁₋₆-Alkoxy, C₁₋₆-Alkoxy-(C₁₋₆)-alkoxy, C₂₋₆-Alkinyloxy, C₃₋₆-Cycloalkyl, Nitro, Cyano, C₁₋₆-Halogenalkoxy, C₂₋₆-Halogenalkenyloxy, S(O)ₚR¹¹, OSO₂R¹², NR¹³SO₂R¹⁴, NR¹⁵R¹⁶, NR¹⁷COR¹⁸, COR¹⁹, SiR²⁰R²¹R²², SCN, gegebenenfalls substituiertes Aryl oder gegebenenfalls substituiertes Heteroaryl oder gegebenenfalls substituiertes Heterocyclyl sind; R¹¹, R¹² und R¹⁴ unabhängig voneinander C₁₋₆-Alkyl, C₁₋₆-Halogenalkyl oder gegebenenfalls substituiertes Aryl sind; R¹³ und R¹⁷ unabhängig voneinander Wasserstoff oder C₁₋₂-Alkyl sind; R¹⁵ und R¹⁶ unabhängig voneinander Wasserstoff oder C₁₋₃-Alkyl sind; oder R¹⁵ und R¹⁶ zusammen mit dem N-Atom, an das sie gebunden sind, einen 5- oder 6-gliedrigen, gegebenenfalls substituierten, heterocyclischen Ring bilden, der ein weiteres Heteroatom enthalten kann, das aus O und S ausgewählt ist; R¹⁸ und R¹⁹ unabhängig voneinander Wasserstoff, C₁₋₆-Alkyl, C₁₋₆-Alkoxy, gegebenenfalls substituiertes Aryl, gegebenenfalls substituiertes Heteroaryl oder NR²³R²⁴ sind; R²⁰, R²¹ und R²² unabhängig voneinander C₁₋₄-Alkyl oder Aryl sind; R²³ und R²⁴ unabhängig voneinander Wasserstoff oder C₁₋₃-Alkyl sind; oder R²³ und R²⁴ zusammen mit dem N-Atom, an das sie gebunden sind, einen 5- oder 6-gliedrigen, gegebenenfalls substituierten, heterocyclischen Ring bilden, der ein weiteres Heteroatom enthalten kann, das aus O und S ausgewählt ist; und p 0, 1 oder 2 ist,
wobei das Verfahren die Umsetzung einer Verbindung der Formel (II): 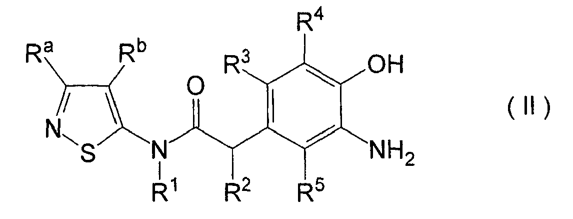 worin R^{a}, R^{b}, R¹, R², R³, R⁴ und R⁵ wie im Hinblick auf Formel (I) definiert sind, mit einer Verbindung der Formel (III):
R^{C}CHO (III)
worin R^{C} wie im Hinblick auf Formel (I) definiert ist, und einem Oxidationsmittel umfasst.

2. Verfahren gemäss Anspruch 1, worin R^{C} eine Gruppe 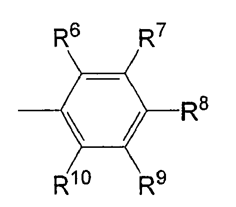 ist, worin R⁶ und R¹⁰ unabhängig voneinander Wasserstoff, Halogen, C₁₋₃-Alkyl, C₁₋₂-Halogenalkyl, C₁₋₂-Alkoxy, Nitro, Cyano, C₁₋₂-Halogenalkoxy, C₁₋₂-Alkylthio, Amino, C₁₋₃-Alkylamino oder Di-(C₁₋₃)-alkylamino sind, vorausgesetzt, dass zumindest eines von R⁶ und R¹⁰ nicht Wasserstoff ist; und R⁷, R⁸ und R⁹ unabhängig voneinander Wasserstoff, Halogen, C₁₋₆-Alkyl, C₂₋₆-Alkenyl, C₂₋₆-Alkinyl, C₁₋₆-Halogenalkyl, C₁₋₆-Alkoxy-(C₁₋₆)-alkyl, C₁₋₆-Alkoxy, C₃₋₆-Cycloalkyl, Nitro, Cyano, C₁₋₆-Halogenalkoxy, S(O)ₚR¹¹, OSO₂R¹², NR¹³SO₂R¹⁴, NR¹⁵R¹⁶, NR¹⁷COR¹⁸, COR¹⁹, SiR²⁰R²¹R²², SCN, gegebenenfalls substituiertes Aryl oder gegebenenfalls substituiertes Heteroaryl.

3. Verfahren gemäss Anspruch 1, worin R^{C} C₁₋₆-Alkyl oder C₁₋₆-Halogenalkyl ist.

4. Verfahren gemäss einem der vorhergehenden Ansprüche, worin R¹ Wasserstoff, C₁₋₂-Alkyl oder (C₁₋₆)-Alkoxymethyl ist.

5. Verfahren gemäss einem der vorhergehenden Ansprüche, worin R² Wasserstoff oder Fluor ist.

6. Verfahren gemäss einem der vorhergehenden Ansprüche, worin R³, R⁴ und R⁵ jeweils unabhängig voneinander Wasserstoff oder Halogen sind.

7. Verfahren zur Herstellung einer Verbindung der Formel (I), wie in Anspruch 1 definiert, wobei das Verfahren die Umsetzung einer Verbindung der Formel (II), wie in Anspruch 1 definiert, mit einer Verbindung der Formel (III), wie in Anspruch 1 definiert, umfasst, um eine Verbindung der Formel (IV) oder Formel (IV') herzustellen: 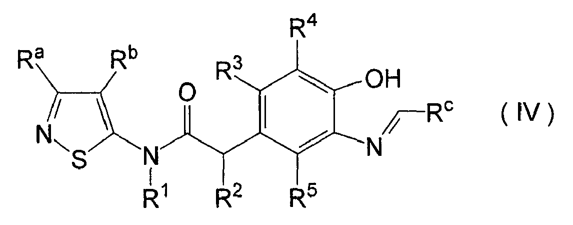 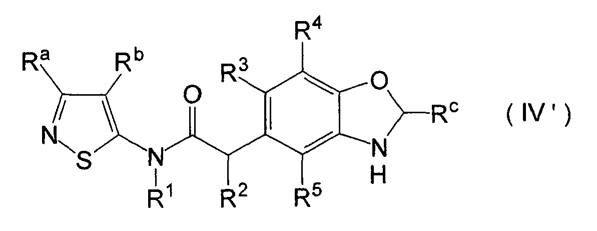 worin R^{a} C₁₋₃-Alkyl ist; R^{b} Halogen ist; R^{C} C₁₋₆-Alkoxy-(C₁₋₆)-alkyl, C₁₋₆-Halogenalkyl, C₁₋₆-Alkyl oder C₁₋₆-Alkoxy oder eine Gruppe 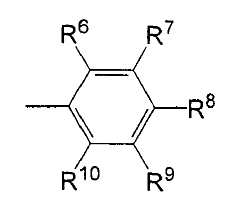 ist; R¹ Wasserstoff, C₁₋₂-Alkyl, (C₁₋₆)-Alkoxymethyl oder Propargyl ist; R² Wasserstoff, Methyl oder Fluor ist; R³, R⁴ und R⁵ unabhängig voneinander Wasserstoff, Halogen, C₁₋₂-Alkyl, C₁₋₂-Alkoxy oder C₁₋₂-Halogenalkyl sind; R⁶ und R¹⁰ unabhängig voneinander Wasserstoff, Halogen, C₁₋₃-Alkyl, C₁₋₂-Halogenalkyl, C₁₋₂-Alkoxy, Nitro, Cyano, C₁₋₂-Halogenalkoxy, C₁₋₈-Alkylthio, C₁₋₆-Alkylsulfinyl, C₁₋₆-Alkylsulfonyl, Amino, C₁₋₃-Alkylamino oder Di-(C₁₋₃)-alkylamino sind; R⁷, R⁸ und R⁹ unabhängig voneinander Wasserstoff, Halogen, C₁₋₆-Alkyl, C₂₋₆-Alkenyl, C₂₋₆-Alkinyl, C₁₋₆-Halogenalkyl, C₁₋₆-Alkoxy-(C₁₋₆)-alkyl, C₁₋₆-Alkoxy, C₁₋₆-Alkoxy-(C₁₋₆)-alkoxy, C₂₋₆-Alkinyloxy, C₃₋₆-Cycloalkyl, Nitro, Cyano, C₁₋₆-Halogenalkoxy, C₂₋₆-Halogenalkenyloxy, S(O)ₚR¹¹, OSO₂R¹², NR¹³SO₂R¹⁴, NR¹⁵R¹⁶, NR¹⁷COR¹⁸, COR¹⁹, SiR²⁰R²¹R²², SCN, gegebenenfalls substituiertes Aryl oder gegebenenfalls substituiertes Heteroaryl oder gegebenenfalls substituiertes Heterocyclyl sind; R¹¹, R¹² und R¹⁴ unabhängig voneinander C₁₋₆-Alkyl, C₁₋₆-Halogenalkyl oder gegebenenfalls substituiertes Aryl sind; R¹³ und R¹⁷ unabhängig voneinander Wasserstoff oder C₁₋₂-Alkyl sind; R¹⁵ und R¹⁶ unabhängig voneinander Wasserstoff oder C₁₋₃-Alkyl sind; oder R¹⁵ und R¹⁶ zusammen mit dem N-Atom, an das sie gebunden sind, einen 5- oder 6-gliedrigen, gegebenenfalls substituierten, heterocyclischen Ring bilden, der ein weiteres Heteroatom enthalten kann, das aus O und S ausgewählt ist; R¹⁸ und _{R}¹⁹ unabhängig voneinander Wasserstoff, C₁-₆-Alkyl, C₁-₆-Alkoxy, gegebenenfalls substituiertes Aryl, gegebenenfalls substituiertes Heteroaryl oder NR²³R²⁴ sind; R²⁰, R²¹ und R²² unabhängig voneinander C₁₋₄-Alkyl oder Aryl sind; R²³ und R²⁴ unabhängig voneinander Wasserstoff oder C₁₋₃-Alkyl sind; oder R²³ und R²⁴ zusammen mit dem N-Atom, an das sie gebunden sind, einen 5- oder 6-gliedrigen, gegebenenfalls substituierten, heterocyclischen Ring bilden, der ein weiteres Heteroatom enthalten kann, das aus O und S ausgewählt ist; und p 0, 1 oder 2 ist,
und Umsetzen der Verbindung der Formeln (IV) oder (IV') mit einem Oxidationsmittel.

8. Verfahren gemäss einem der vorhergehenden Ansprüche, worin das Oxidationsmittel UV-Licht; Sauerstoff; Brom; Acetate, wie z.B. Bleitetraacetat, Iodosobenzoldiacetat und Mangantriacetat; Perchlorate, wie z.B. Natriumperchlorat und Thianthrenradikalkation-Perchlorat; Manganate, wie z.B. Bariummanganat; Peroxide, wie z.B. Nickelperoxid; Oxide, wie z.B. Mangandioxid; Dichlor-5,6-dicyano-1,4-benzochinon und N-Bromsuccinimid, ist.

9. Verbindung der Formel (IV) oder (IV'): 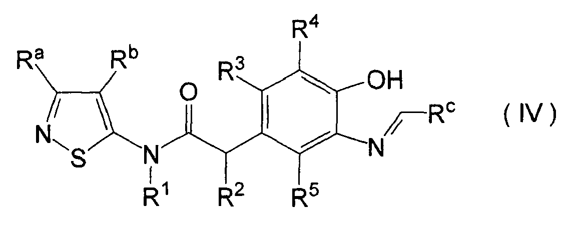 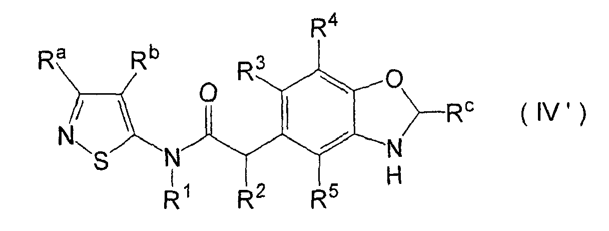 worin R^{a} C₁₋₃-Alkyl ist; R^{b} Halogen ist; R^{c} C₁₋₆-Alkoxy-(C₁₋₆)-alkyl, C₁₋₆-Halogenalkyl, C₁₋₆-Alkyl oder C₁₋₆-Alkoxy oder eine Gruppe 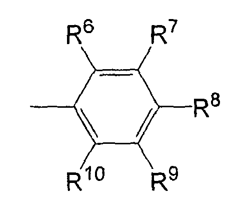 ist; R¹ Wasserstoff, C₁₋₂-Alkyl, (C₁₋₆)-Alkoxymethyl oder Propargyl ist; R² Wasserstoff, Methyl oder Fluor ist; R³, R⁴ und R⁵ unabhängig voneinander Wasserstoff, Halogen, C₁₋₂-Alkyl, C₁₋₂-Alkoxy oder C₁₋₂-Halogenalkyl sind; R⁶ und R¹⁰ unabhängig voneinander Wasserstoff, Halogen, C₁₋₃-Alkyl, C₁₋₂-Halogenalkyl, C₁₋₂-Alkoxy, Nitro, Cyano, C₁₋₂-Halogenalkoxy, C₁₋₈-Alkylthio, C₁₋₆-Alkylsulfinyl, C₁₋₆-Alkylsulfonyl, Amino, C₁₋₃-Alkylamino oder Di-(C₁₋₃)-alkylamino sind; R⁷, R⁸ und R⁹ unabhängig voneinander Wasserstoff, Halogen, C₁₋₆-Alkyl, C₂₋₆-Alkenyl, C₂₋₆-Alkinyl, C₁₋₆-Halogenalkyl, C₁₋₆-Alkoxy-(C₁₋₆)-alkyl, C₁₋₆-Alkoxy, C₁₋₆-Alkoxy-(C₁₋₆)-alkoxy, C₂₋₆-Alkinyloxy, C₃₋₆-Cycloalkyl, Nitro, Cyano, C₁₋₆-Halogenalkoxy, C₂₋₆-Halogenalkenyloxy, S(O)ₚR¹¹, OSO₂R¹², NR¹³SO₂R¹⁴, NR¹⁵R¹⁶, NR¹⁷COR¹⁸, COR¹⁹, SiR²⁰R²¹R²², SCN, gegebenenfalls substituiertes Aryl oder gegebenenfalls substituiertes Heteroaryl oder gegebenenfalls substituiertes Heterocyclyl sind; R¹¹, R¹² und R¹⁴ unabhängig voneinander C₁₋₆-Alkyl, C₁₋₆-Halogenalkyl oder gegebenenfalls substituiertes Aryl sind; R¹³ und R¹⁷ unabhängig voneinander Wasserstoff oder C₁₋₂-Alkyl sind; R¹⁵ und R¹⁶ unabhängig voneinander Wasserstoff oder C₁₋₃-Alkyl sind; oder R¹⁵ und R¹⁶ zusammen mit dem N-Atom, an das sie gebunden sind, einen 5- oder 6-gliedrigen, gegebenenfalls substituierten, heterocyclischen Ring bilden, der ein weiteres Heteroatom enthalten kann, das aus O und S ausgewählt ist; R¹⁸ und R¹⁹ unabhängig voneinander Wasserstoff, C₁₋₆-Alkyl, C₁₋₆-Alkoxy, gegebenenfalls substituiertes Aryl, gegebenenfalls substituiertes Heteroaryl oder NR²³R²⁴ sind; R²⁰, R²¹ und R²² unabhängig voneinander C₁₋₄-Alkyl oder Aryl sind; R²³ und R²⁴ unabhängig voneinander Wasserstoff oder C₁₋₃-Alkyl sind; oder R²³ und R²⁴ zusammen mit dem N-Atom, an das sie gebunden sind, einen 5- oder 6-gliedrigen, gegebenenfalls substituierten, heterocyclischen Ring bilden, der ein weiteres Heteroatom enthalten kann, das aus O und S ausgewählt ist; und p 0, 1 oder 2 ist.

## Revendications

1. Procédé de préparation d'un composé de formule (I) 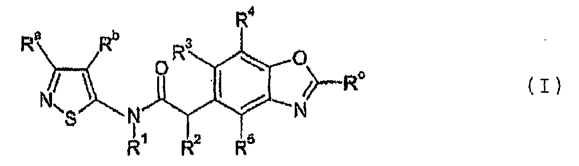 où R^{a} est un alkyle en C₁₋₃ ; R^{b} est un halogène ; R^{c} est un alcoxy en C₁₋₆-alkyle en (C₁₋₆), un haloalkyle en C₁₋₆, un alkyle en C₁₋₆ ou alcoxy en C₁₋₆, ou est un groupe 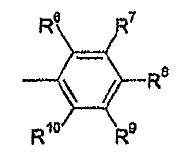 R¹ est un hydrogène, un alkyle en C₁₋₂, un alcoxyméthyle en (C₁₋₆) ou propargyle, R² est un hydrogène, méthyle ou fluoro ; R³, R⁴ et R⁵ sont, indépendamment, un hydrogène, un halogène, un alkyle en C₁₋₂, un alcoxy en C₁₋₂ ou un haloalkyle en C₁₋₂ ; R⁶ et R¹⁰ sont, indépendamment, un hydrogène, un halogène, un alkyle en C₁₋₃, un haloalkyle en C₁₋₂, un alcoxy en C₁₋₂, un nitro, un cyano, un haloalcoxy en C₁₋₂, un alkylthio en C₁₋₈, un alkylsulfinyle en C₁₋₆, un alkylsulfonyle en C₁₋₆, un amino, un alkylamino en C₁₋₃ ou un di-alkylamino en (C₁₋₃) ; R⁷, R⁸ et R⁹ sont, indépendamment, un hydrogène, un halogène, un alkyle en C₁₋₆, un alcényle en C₂₋₆, un alcynyle en C₂₋₆, un haloalkyle en C₁₋₆, un alcoxy en C₁₋₆-alkyle en (C₁₋₆), un alcoxy en C₁₋₆, un alcoxy en C₁₋₆-alcoxy en (C₁₋₆), un alcynyloxy en C₂₋₆, un cycloalkyle en C₃₋₆, un nitro, un cyano, un haloalcoxy en C₁₋₆, un haloalcényloxy en C₂₋₆, un S(O)ₚR¹¹, un OSO₂R¹², un NR¹³SO₂R¹⁴, un NR¹⁵R¹⁶, NR¹⁷COR¹⁸, COR¹⁹, SiR²⁰R²¹R²², SCN, un aryle éventuellement substitué ou un hétéroaryle éventuellement substitué ou un hétérocyclyle éventuellement substitué ;
R¹¹, R¹² et R¹⁴ sont, indépendamment, un alkyle en C₁₋₆, un haloalkyle en C₁₋₆, ou un aryle éventuellement substitué ; R¹³ et R¹⁷ sont, indépendamment, un hydrogène ou un alkyle en C₁₋₂ ; ou R¹⁵ et R¹⁶ sont, indépendamment, un hydrogène ou un alkyle en C₁₋₃ ; ou R¹⁵ et R¹⁶ ensemble avec l'atome de N auquel ils sont fixés forment un cycle hétérocyclique à cinq ou six éléments éventuellement substitué qui peut contenir un autre hétéroatome choisi parmi un O et S ; R¹⁸ et R¹⁹ sont, indépendamment, un hydrogène, un alkyle en C₁₋₆, un alcoxy en C₁₋₆, un aryle éventuellement substitué, un hétéroaryle éventuellement substitué, ou un NR²³R²⁴ ; R²⁰, R²¹ et R²² sont, indépendamment, un alkyle ou aryle en C₁₋₄ ;
R²³ et R²⁴ sont, indépendamment, un hydrogène ou un alkyle en C₁₋₃ ; ou R²³ et R²⁴, ensemble avec l'atome N auquel ils sont fixés, forment un cycle hétérocyclique éventuellement substitué à cinq ou six éléments qui peut contenir un autre hétéroatome choisi parmi O et S ; et p est égal à 0, 1 ou 2, le procédé comprenant la réaction d'une formule de composé II 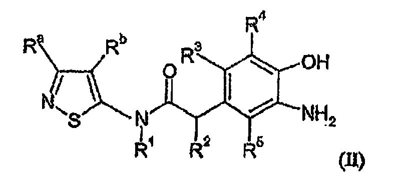 où R^{a}, R^{b}, R¹, R², R³, R⁴ et R⁵ sont comme définis relativement à la formule (I) avec un composé de formule III
R^{c}CHO (III)
où R^{c} est comme défini relativement à la formule (I) et un agent oxydant.

2. Procédé selon la revendication 1, dans lequel R^{c} est un groupe 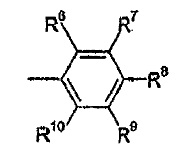 où R⁶ et R¹⁰ sont, indépendamment, un hydrogène, un halogène, un alkyle en C₁₋₃, un haloalkyle en C₁₋₂, un alcoxy en C₁₋₂, un nitro, un cyano, un haloalcoxy en C₁₋₂, un alkylthio en C₁₋₂, un amino, un alkylamino en C₁₋₃, ou un di-alkylamino en (C₁₋₃), à condition qu'au moins un élément parmi R⁶ et R¹⁰ ne soit pas un hydrogène ; et R⁷, R⁸ et R⁹ sont indépendamment un hydrogène, un halogène, un alkyle en C₁₋₆, un alcényle en C₂₋₆, un alcynyle en C₂₋₆, un haloalkyle en C₁₋₆, un alcoxy en C₁₋₆-alkyle en (C₁₋₆), un alcoxy en C₁₋₆, un cycloalkyle en C₃₋₆, un nitro, un cyano, un haloalcoxy en C₁₋₆, un S(O)ₚR¹¹, un OSO₂R¹², NR¹³SO₂R¹⁴, NR¹⁵R¹⁶, NR¹⁷COR¹⁸, COR¹⁹, SiR²⁰R²¹R²², SCN, un aryle éventuellement substitué ou un hétéroaryle éventuellement substitué.

3. Procédé selon la revendication 1, dans lequel R^{c} est un alkyle en C₁₋₆ ou un haloalkyle en C₁₋₆.

4. Procédé selon l'une quelconque des revendications précédentes, dans lequel R¹ est un hydrogène, un alkyle en C₁₋₂ ou un alcoxyméthyle en (C₁₋₆).

5. Procédé selon l'une quelconque des revendications précédentes, dans lequel R² est un hydrogène ou un fluoro.

6. Procédé selon l'une quelconque des revendications précédentes, dans lequel R³, R⁴, et R⁵ sont chacun, indépendamment, un hydrogène ou un halogène.

7. Procédé de préparation d'un composé de formule I selon la revendication 1, ledit procédé comprenant la réaction d'un composé de formule II, comme défini dans la revendication 1, avec un composé de formule III comme défini dans la revendication 1, afin de produire un composé de formule (IV) ou de formule (IV') 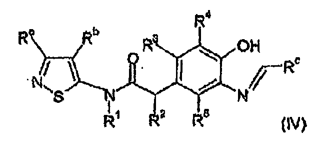 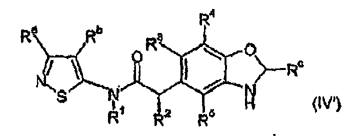 où R^{a} est un alkyle en C₁₋₃ ; R^{b} est un halogène ; R^{c} est un alcoxy en C₁₋₆-alkyle en (C₁₋₆), un haloalkyle en C₁₋₆, un alkyle en C₁₋₆, ou un alcoxy en C₁₋₆ ou est un groupe 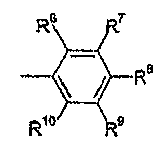 R¹ est un hydrogène, un alkyle en C₁₋₂, un alcoxyméthyle en (C₁₋₆) ou propargyle, R² est un hydrogène, méthyle ou fluoro ; R³, R⁴ et R⁵ sont, indépendamment, un hydrogène, un halogène, un alkyle en C₁₋₂, un alcoxy en C₁₋₂ ou un haloalkyle en C₁₋₂ ; R⁶ et R¹⁰ sont, indépendamment, un hydrogène, un halogène, un alkyle en C₁₋₃, un haloalkyle en C₁₋₂, un alcoxy en C₁₋₂, un nitro, un cyano, un haloalcoxy en C₁₋₂, un alkylthio en C₁₋₈, un alkylsulfinyle en C₁₋₆, un alkylsulfonyle en C₁₋₆, un amino, un alkylamino en C₁₋₃ ou un di-alkylamino en (C₁₋₃) ; R⁷, R⁸ et R⁹ sont, indépendamment, un hydrogène, un halogène, un alkyle en C₁₋₆, un alcényle en C₂₋₆, un alcynyle en C₂₋₆, un haloalkyle en C₁₋₆, un alcoxy en C₁₋₆-alkyle en (C₁₋₆), un alcoxy en C₁₋₆, un alcoxy en C₁₋₆-alcoxy en (C₁₋₆), un alcynyloxy en C₂₋₆, un cycloalkyle en C₃₋₆, un nitro, un cyano, un haloalcoxy en C₁₋₆, un haloalcényloxy en C₂₋₆, un S(O)ₚR¹¹, un OSO₂R¹², un NR¹³SO₂R¹⁴, un NR¹⁵R¹⁶, NR¹⁷COR¹⁸, COR¹⁹, SiR²⁰R²¹R²², SCN, un aryle éventuellement substitué ou un hétéroaryle éventuellement substitué ou un hétérocyclyle éventuellement substitué ;
R¹¹, R¹² et R¹⁴ sont, indépendamment, un alkyle en C₁₋₆, un haloalkyle en C₁₋₆, ou un aryle éventuellement substitué ; R¹³ et R¹⁷ sont, indépendamment, un hydrogène ou un alkyle en C₁₋₂ ; R¹⁵ et R¹⁶ sont, indépendamment, un hydrogène ou un alkyle en C₁₋₃ ; ou R¹⁵ et R¹⁶ ensemble avec l'atome de N auquel ils sont fixés forment un cycle hétérocyclique à cinq ou six éléments, éventuellement substitué qui peut contenir un autre hétéroatome choisi parmi un O et S ; R¹⁸ et R¹⁹ sont, indépendamment, un hydrogène, un alkyle en C₁₋₆, un alcoxy en C₁₋₆, un aryle éventuellement substitué, un hétéroaryle éventuellement substitué, ou un NR²³R²⁴ ; R²⁰, R²¹ et R²² sont, indépendamment, un alkyle ou aryle en C₁₋₄ ;
R²³ et R²⁴ sont, indépendamment, un hydrogène ou un alkyle en C₁₋₃ ; ou R²³ et R²⁴, ensemble avec l'atome N auquel ils sont fixés, forment un cycle hétérocyclique éventuellement substitué à cinq ou six éléments qui peut contenir un autre hétéroatome choisi parmi O et S ; et p est égal à 0, 1 ou 2 et la réaction du composé de formule (IV) ou (IV') avec un agent oxydant.

8. Procédé selon l'une quelconque des revendications précédentes, dans lequel l'agent oxydant est une lumière UV, l'air, l'oxygène, le brome, les acétates comme le tétraacétate de plomb, l'iodosobenzènediacétate, et le triacétate de manganèse, les perchlorates comme le perchlorate de sodium, et le perchlorate de radical de cation thianthrène, les manganates comme le manganate de baryum, les peroxydes comme le peroxyde de nickel, les oxydes comme le dioxyde de manganèse, la dichloro-5,6-dicyano-1,4-benzoquinone et le N-bromosuccinimide.

9. Composé de formule IV ou de formule IV' 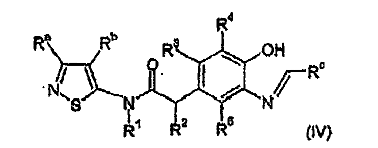 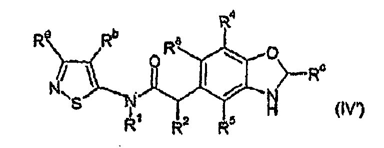 où R^{a} est un alkyle en C₁₋₃ ; R^{b} est un halogène ; R^{c} est un alcoxy en C₁₋₆-alkyle en (C₁₋₆), un haloalkyle en C₁₋₆, un alkyle en C₁₋₆, ou un alcoxy en C₁₋₆ ou est un groupe 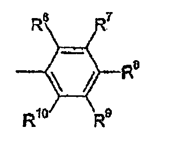 R¹ est un hydrogène, un alkyle en C₁₋₂, un alcoxyméthyle en (C₁₋₆) ou propargyle, R² est un hydrogène, méthyle ou fluoro ; R³, R⁴ et R⁵ sont, indépendamment, un hydrogène, un halogène, un alkyle en C₁₋₂, un alcoxy en C₁₋₂ ou un haloalkyle en C₁₋₂ ; R⁶ et R¹⁰ sont, indépendamment, un hydrogène, un halogène, un alkyle en C₁₋₃, un haloalkyle en C₁₋₂, un alcoxy en C₁₋₂, un nitro, un cyano, un haloalcoxy en C₁₋₂, un alkylthio en C₁₋₈, un alkylsulfinyle en C₁₋₆, un alkylsulfonyle en C₁₋₆, un amino, un alkylamino en C₁₋₃ ou un di-alkylamino en (C₁₋₃) ; R⁷, R⁸ et R⁹ sont, indépendamment, un hydrogène, un halogène, un alkyle en C₁₋₆, un alcényle en C₂₋₆, un alcynyle en C₂₋₆, un haloalkyle en C₁₋₆, un alcoxy en C₁₋₆-alkyle en (C₁₋₆), un alcoxy en C₁₋₆, un alcoxy en C₁₋₆-alcoxy en (C₁₋₆), un alcynyloxy en C₂₋₆, un cycloalkyle en C₃₋₆, un nitro, un cyano, un haloalcoxy en C₁₋₆, un haloalcényloxy en C₂₋₆, un S(O)ₚR¹¹, un OSO₂R¹², un NR¹³SO₂R¹⁴, un NR¹⁵R¹⁶, NR¹⁷COR¹⁸, COR¹⁹, SiR²⁰R²¹R²² SCN, un aryle éventuellement substitué ou un hétéroaryle éventuellement substitué ou un hétérocyclyle éventuellement substitué ;
R¹¹, R¹² et R¹⁴ sont, indépendamment, un alkyle en C₁₋₆, un haloalkyle en C₁₋₆, ou un aryle éventuellement substitué ; R¹³ et R¹⁷ sont, indépendamment, un hydrogène ou un alkyle en C₁₋₂ ; R¹⁵ et R¹⁶. sont, indépendamment, un hydrogène ou un alkyle en C₁₋₃ ; ou R¹⁵ et R¹⁶ ensemble avec l'atome de N auquel ils sont fixés forment un cycle hétérocyclique à cinq ou six éléments, éventuellement substitué qui peut contenir un autre hétéroatome choisi parmi un O et S ; R¹⁸ et R¹⁹ sont, indépendamment, un hydrogène, un alkyle en C₁₋₆, un alcoxy en C₁₋₆, un aryle éventuellement substitué, un hétéroaryle éventuellement substitué, ou un NR²³R²⁴ ; R²⁰, R²¹ et R²² sont, indépendamment, un alkyle ou aryle en C₁₋₄;
R²³ et R²⁴ sont, indépendamment, un hydrogène ou un alkyle en C₁₋₃ ; ou R²³ et R²⁴, ensemble avec l'atome N auquel ils sont fixés, forment un cycle hétérocyclique éventuellement substitué à cinq ou six éléments qui peut contenir un autre hétéroatome choisi parmi O et S ; et p est égal à 0, 1 ou 2.
